Europäisches Patentamt

European Patent Office

Office européen des brevets

(11) Publication number: **0 201 800 B1**

(12) **EUROPEAN PATENT SPECIFICATION**

(45) Date of publication of patent specification: **25.09.91**  (51) Int. Cl.5: **C12N 5/06, C07J 9/00**

(21) Application number: **86105904.6**

(22) Date of filing: **29.04.86**

(54) **A cholesterol-rich fraction, process therefor and use thereof in cell culture.**

(30) Priority: **10.05.85 US 733303**
**10.05.85 US 732856**

(43) Date of publication of application:
**20.11.86 Bulletin 86/47**

(45) Publication of the grant of the patent:
**25.09.91 Bulletin 91/39**

(84) Designated Contracting States:
**AT BE CH DE FR GB IT LI NL SE**

(56) References cited:
**US-A- 4 290 774**
**US-A- 4 403 042**
**US-A- 4 582 807**

**CHEMICAL ABSTRACTS, vol. 92, no. 9, 3rd March 1980, page 471, abstract no. 74037p, Columbus, Ohio, US; Y. HONMA et al.: "Replacement of serum by insulin, transfer-rip, albumin, phosphatidyl choline, choles-terol, and some trace elements in cultures of mouse myeloid leukemia cells sensitive to inducers of differentiation", & EXP. CELL RES. 1979, 124(2), 421-8**

(73) Proprietor: **MILES INC.**
**1127 Myrtle Street**
**Elkhart Indiana 46514(US)**

(72) Inventor: **Girgis, Makram Melek**
**631 Brookmont Blvd.**
**Bradley, IL.(US)**
Inventor: **Montalto, Joseph Gaspare**
**1008 Mallard**
**Bradley, IL.(US)**
Inventor: **Pieczynski, William John**
**26W587 Grand Ave.**
**Wheaton, IL.(US)**
Inventor: **Thomas, Jean Violet**
**1417 W. Hawkins**
**Kankakee, IL.(US)**
Inventor: **Tihon, Claude**
**1177 Kenilworth**
**Naperville, IL.(US)**

(74) Representative: **Dänner, Klaus, Dr. et al**
**c/o Bayer AG Konzernverwaltung RP Patente Konzern**
**W-5090 Leverkusen 1 Bayerwerk(DE)**

CHEMICAL ABSTRACTS, vol. 102, no. 19, 13th May 1985, page 429, abstract no. 164540b, Columbus, Ohio, US; J. SATO et al.: " Cholesterol requirement of NS-1 mouse myeloma cells for growth in serum-free medium", & MOL. BIOL. MED. 1984 2(2), 121-34

## Description

### BACKGROUND AND PRIOR ART

This invention relates to the isolation and purification of a cholesterol-rich fraction from mammalian blood plasma or serum which is useful as a growth medium ingredient, especially in cell culture.

It is known that cholesterol and cholesterol-containing fractions obtained from bovine serum are useful to promote the growth of various organisms. J.Bacteriol., Vol. 135, pp. 818-827 (1978) describes the use of a cholesterol-containing bovine serum fraction in the growth of Mycoplasma pneumoniae and Mycoplasma arthritidis. J.Gen.Microbiology, Vol. 116, pp. 539-543 (1980) describes the use of USP cholesterol in the growth of Treponema hyodysenteriae. U.S. Patent No. 4,290,774 describes the production of a specific cholesterol-rich fraction from mammalian plasma or serum by an overall process which involves the step of treatment with an alkaline carbonate and an alkaline earth salt to precipitate denatured proteins and separate them from the soluble cholesterol. The resulting product is useful primarily as a cholesterol reference material. This particular cholesterol concentrate can also be used in the detection of cell membrane antigens and corresponding antibodies as set forth in U.S. Patent No. 4,403,042. Zeit.Klin.Chem. 6(3), pp. 186-190 (1968) describes the removal of certain lipoproteins from human serum by use of colloidal silicic acid. None of the above prior art discloses or suggests the specific process of the present application or the specific cholesterol-rich fraction produced thereby.

Various mammalian cells are grown, for example, to screen the effects of chemical substances on such cells, to produce cells for cancer research, and to produce metabolites and antibodies for subsequent research, diagnostic or therapeutic purposes. These mammalian cells are usually grown in an appropriate nutrient medium containing 10-15 volume percent fetal calf serum. This serum level has been found generally essential for proper cell growth under such cell culture conditions. Cancer Research, Vol. 41, pp. 473-477 (Feb. 1981) is a typical prior art reference describing use of 10 percent fetal calf serum to grow and maintain mammalian cancer cells. Experimental Cell Research, Vol. 131, pp. 31-40 (1981) discloses use of 15 percent fetal calf serum to maintain mammalian cells.

While the use of fetal calf serum is desirable for proper cell growth, it has several disadvantages. First, it is a relatively expensive material, and its use greatly increases the cost of cell culture. Second, it is difficult to get serum with consistent growth characteristics. Third, the serum in the nutrient media interferes with the purification and recovery of the desired proteins or glycoproteins secreted by the cells into the growth medium. When the fetal calf serum content is significantly reduced to minimize the above disadvantages, the cell growth is dramatically cut back.

It is known that lipoproteins, such as cholesterol, may have some desirable effects on cell growth. In Vitro, Vol. 17, No. 5, pp. 519-530 (1981) discloses that mixtures of high density lipoproteins and transferrin can be used to grow certain mammalian cells in the absence of serum. The above Cancer Research article also discusses the cholesterol metabolism of cancer cells in a medium containing 10 percent fetal calf serum. The above Experimental Cell Research article also discusses cellular cholesterol biosynthesis.

None of the above prior art discloses or suggests the use of a cholesterol-rich fraction produced by a process different from the prior art to enable a satisfactory amount of cell growth to be achieved at a significantly reduced level of fetal calf serum and even in a serum-free medium.

### SUMMARY OF THE INVENTION

In accordance with the present invention, a process is provided for isolating and purifying a cholesterol-rich fraction from mammalian blood plasma or serum or fraction thereof containing cholesterol which comprises the steps of:

(a) contacting a liquid cholesterol-containing plasma or serum or fraction thereof with a silica adsorbent to adsorb the cholesterol;

(b) separating the adsorbed cholesterol from the remaining liquid plasma or serum;

(c) freezing and thawing the adsorbed cholesterol;

(d) eluting the adsorbed cholesterol at a pH from 9.0 to 11.5;

(e) concentrating the eluted cholesterol solution by ultrafiltration;

(f) adjusting the pH of the concentrated cholesterol solution to a value in the range from 11.0 to 11.4;

(g) dialyzing the concentrated cholesterol solution sequentially against sodium carbonate and water;

(h) further concentrating the dialyzed cholesterol solution by ultrafiltration to desired cholesterol level;

(i) adjusting the pH of the concentrated cholesterol solution to a value in the range from 7.0 to 11.0;

(j) heating the concentrated cholesterol solution at 50° to 100° C. for 30 minutes to 24 hours; and

(k) recovering therefrom a purified cholesterol-rich fraction.

The cholesterol-rich fraction produced by this process is a new material which is especially useful as a nutrient medium ingredient for the cell culture growth of mammalian cells whereby suitable cell yield is obtained with significant reduction in use of fetal calf serum and even elimination of fetal calf serum.

## DESCRIPTION OF THE INVENTION

The starting material for use in the present invention can be any mammalian blood plasma or serum fraction containing cholesterol. Suitable starting materials can be bovine, horse, sheep, pig or human plasma or serum or fraction thereof that contains cholesterol, such a fibrinogen-poor plasma, Cohn Fraction I supernatant, an ammonium sulfate supernatant rich in lipoprotein and the like. The preferred starting material is bovine serum. If the starting material is serum, it is preferred to add a soluble salt, such as sodium citrate, to an ionic strength of 0.25 to 1.0. Other suitable salts include sodium chloride, sodium phosphate, potassium phosphate, ammonium sulfate and sodium sulfate. The addition of a soluble salt to the above concentration will increase the amount of cholesterol adsorbed in the subsequent silica adsorption step. Bovine or human plasma, for example, is normally collected by a method which includes addition of citrate as an anti-coagulant. This salt concentration is usually sufficient for the adsorption step and no additional salt is needed.

The plasma or serum starting material is maintained at a temperature of from 0° C. to 50° C., preferably from 20° C. to 25° C. The pH is adjusted to a range of from 5.5 to 9.0, preferably from 7.0 to 8.0.

The silica adsorbent useful in this invention does not have a critical composition. Appropriate silica materials are the microfine silica available under the trademark Cabosil from Cabot Corporation and the powdered silica available under the trademark Aerosil 380 from Cary Company. The silica is added to the liquid plasma or serum in an amount of 1 to 50 g/l., preferably from 10 to 20 g/l.

The silica suspension in the liquid plasma or serum is then mixed for about 3 to 4 hours. The silica containing adsorbed cholesterol is then separated from the remaining liquid plasma or serum preferably by centrifugation, and the liquid phase is discarded. The silica paste is then frozen at -20° C. and held at this temperature for at least one week and preferably two weeks. The frozen paste is then thawed to room temperature (about 20° - 25° C.) for 24 to 48 hours. Any liquid that is expressed from the thawed paste is discarded.

The silica paste is washed to remove any undesirable proteins. This is accomplished by suspending the paste in an aqueous salt solution containing abut 0.15M sodium chloride. Other useful salts are sodium acetate and sodium phosphate. The salt solution is used in an amount about twice the weight of the paste. The paste is separated from the liquid. This washing procedure using a salt solution is preferably repeated two times to remove occluded proteins.

The washed paste is suspended in about 2 volumes of deionized or distilled water and the pH is adjusted to 9.0 to 11.5, preferably 10.4 to 10.6, by the addition of appropriate alkaline material, such as aqueous sodium hydroxide. The suspension is stirred for about 2 hours during which time the pH is maintained at the desired level by periodic additions of the above alkaline material. This treatment elutes the desired cholesterol fraction from the silica. The suspension is then allowed to settle for 12 to 24 hours, preferably 12-18 hours. The supernatant containing the cholesterol is siphoned off for further treatment. It is preferred to use only this first elution for production of the desired cholesterol-rich fraction product. However, it is possible to repeat the above alkaline suspension, elution, stirring and settling steps two more times and pool the supernatants from the second and third elutions with the first elution material. The silica is discarded.

The cholesterol solution is clarified by filtration and centrifugation to remove any traces of silica and then concentrated to 15 to 50 percent, preferably 20 percent, of its initial volume, by ultrafiltration techniques. The pH is adjusted to a value in the range from 11.0 to 11.4, preferably pH 11.2, by alkaline addition. This assists in the solubilization of any residual silica for subsequent removal.

The cholesterol concentrate is then treated to remove substantially all the salt present. The preferred salt removal technique is to dialyze sequentially against sodium carbonate and water. This salt removal step is important because of the subsequent heat treatment step. Any significant amount of salt will cause undesirable denaturation of the cholesterol upon heating.

The dialyzed cholesterol solution is further concentrated by ultrafiltration to a cholesterol level of 50 to 3000 mg/dl, preferably 1000 to 2000 mg/dl.

The pH of the concentrated cholesterol solution is then adjusted to a value in the range from 7.0 to 11.0, preferably pH 7.6, in order to render it most compatible for subsequent use in a growth medium.

The solution is heated to 50° to 100° C., preferably 80° C., for 30 minutes to 24 hours, preferably 30 minutes to 60 minutes, in order to increase the storage stability of the cholesterol.

The solution is cooled to room temperature and is sterile filtered to recover a purified cholesterol-rich fraction. This product is not pure cholesterol, but it is mixed with minor amounts of unidentified materials which passed through the production process.

The mammalian cells useful in the cell culture process employing as a nutrient medium ingredient the cholesterol-rich fraction of the present invention can be of a wide variety of cells known to be employed in cell culture. Illustrative examples are mouse fibroblast cells, baby hamster kidney cells, mouse myeloma cells, bovine vascular endothelial cells, epidermoid cervical cancer cells, endometrial adenocarcinoma cells, hamster lung cells, and the like. Particularly useful cells for screening purposes are mouse fibroblast cells, baby hamster kidney cells and mouse myeloma cells obtained from the American Type Culture Collection under the designations L929, BHK-21, and P3x63-Ag8.653, respectively.

The cells are grown in the nutrient media under conditions well known for cell culture growth.

The nutrient media employed to grow the above cells are well known in the art. Examples are Dulbecco's Modified Eagle Medium (DME), Eagle's Minimum Essential Medium (MEM), Eagle's Basal Medium (BME), Ham's Medium F-10, Ham's Medium F-12, RPMI 1640 Medium, and the like. These nutrient media are commercially available from M.A.Bioproducts and Sigma Chemical Co., for example. The overall nutrient media also generally contain a buffer, such as HEPES (N-2-hydroxyethyl-piperasine-N'-2-ethanesulfonic acid). The nutrient media can be free of serum or can preferably contain from 0.5 to 2.5 volume percent fetal calf serum. If a serum-free medium is employed, it should contain well-known ingredients, such as pyrogen-free water, HEPES buffer, Dulbecco's Modified Eagle Medium, Ham's Medium F-12, insulin, transferrin, testosterone, sodium selenite, ethanolamine, saturated and unsaturated fatty acids and stabilizing proteins. Such serum-free media are available from several commercial sources. One exemplary product is HL-1 available from Ventrex Laboratories, Inc. The nutrient media also contain from 0.0005 to 0.01 percent (weight/volume basis) cholesterol which is calculated from the cholesterol content of the specific cholesterol-rich fraction. The media preferably contain from 0.001 to 0.002 percent (weight/volume basis) cholesterol which is calculated from the cholesterol content of the specific cholesterol-rich fraction.

The invention will be further described in the following Examples.

EXAMPLE 1
___ _

Fresh bovine serum was brought to a temperature of 20° - 25° C. and 14.7 g/l of sodium citrate (serum ionic strength of 0.5) was added. The resulting solution was agitated for 30 minutes, and the pH was adjusted to 7.0 by addition of an appropriate amount of 1M sodium hydroxide. Finely-divided silica was added in an amount of 10 g/l and the resulting slurry was agitated for 3 hours at room temperature. The silica containing adsorbed material was then separated from the liquid phase by centrifugation, and the liquid was discarded. The silica paste was then frozen at -20° C. and stored at that temperature for 2 weeks. The frozen paste was then thawed at room temperature for 48 hours. The expressed liquid was discarded. The silica paste was then suspended in 2 volumes of 0.85 percent (weight/volume basis) aqueous sodium chloride solution (0.146M NaCl). It was mixed gently for 15 minutes and allowed to settle for at least 3 hours. The supernatant liquid was siphoned off and discarded. This washing step was repeated two times. The washed paste was then suspended in 2 volumes of deionized water. The pH was adjusted to 10.5 with addition of 1M sodium hydroxide. The resulting suspension was stirred at room temperature for 2 hours while readjusting pH to 10.5. The stirring was stopped, and the suspension was allowed to settle for 18 hours. The supernatant was removed by siphon and clarified by filtration and centrifugation. The silica was discarded. The clarified solution was then concentrated to 20 percent of its initial volume by ultrafiltration. The pH of the concentrated material was adjusted to 11.2 by addition of 1M sodium hydroxide. The concentrated material was then dialysed against 6 volumes of 0.01M sodium carbonate at pH 11.2. It was then dialyzed against 6 volumes of deionized water. The cholesterol level of the concentrate was analyzed by known techniques and further concentrated by ultrafiltration to a cholesterol level of 1000 mg./dl. The pH was then adjusted to 7.6 by addition of 1M hydrochloric acid, and the resulting solution was heated at 80° C. for 1 hour. The solution was then cooled to room temperature and sterile filtered. The filtered material was then recovered as a purified cholesterol-rich fraction.

EXAMPLE 2

Five (5) 35 mm culture dishes each containing 3 ml. of Dulbecco's MEM medium commercially available from M.A. Bioproducts buffered at pH 7.2 by 25 mM HEPES and also containing 0.5 volume percent fetal calf serum were each separately plated with 5 x 10⁴ mouse fibroblast cells (ATCC L929). Five (5) similar dishes each containing 3 ml. of the same overall medium described above and also containing 20 micrograms cholesterol (calculated from the cholesterol content of the specific cholesterol-rich fraction prepared in Example 1 above) per ml. of the initial nutrient medium (0.002 percent on a weight/volume basis) were each separately plated with $5 \times 10^4$ cells of the above same mouse cell line. All of the plated dishes were incubated for 6 days at 37° C. The total cells in each of the dishes were counted, and an arithmetical average total cell count for each group of 5 dishes was calculated. The medium containing the specific cholesterol-rich fraction had an average total cell concentration of $3 \times 10^5$ while the medium without the cholesterol had an average total cell concentration of $2 \times 10^4$.

EXAMPLE 3

Five (5) 35 mm culture dishes each containing 3 ml of the fetal calf serum medium described in Example 2 above and also containing 5 micrograms cholesterol (calculated from the cholesterol content of the specific cholesterol-rich fraction prepared in Example 1 above) per ml. of the initial nutrient medium (0.0005 percent on weight/volume basis) were each separately plated with $5 \times 10^4$ mouse fibroblast cells (ATCC L929). Five (5) similar dishes each containing 3 ml. of the same medium and also containing 40 micrograms cholesterol (calculated from the cholesterol content of the specific cholesterol-rich fraction prepared in Example 1 above) per ml. of the initial nutrient medium (0.004 percent on weight/volume basis) were each separately plated with $5 \times 10^4$ cells of the above same mouse cell line. All of the plated dishes were incubated for 6 days at 37° C. The medium containing the smaller amount of added cholesterol had an average total cell concentration of $8 \times 10^4$, while the medium containing the larger amount of added cholesterol had an average total cell concentration of $1.3 \times 10^5$.

EXAMPLE 4

Five (5) 35 mm. culture dishes each containing 3 ml of the fetal calf serum medium described in Example 2 above and also containing 20 micrograms cholesterol (calculated from the cholesterol content of a cholesterol concentrate prepared according to U.S. Patent No. 4,290,774) per ml. of the initial nutrient medium were each separately plated with $5 \times 10^4$ mouse fibroblast cells (ATCC L929). Five (5) similar dishes each containing 3 ml. of the same medium and also containing 20 micrograms cholesterol (calculated from the cholesterol content of a cholesterol concentrate solution obtained commercially from Irvine Scientific) per ml. of the initial nutrient medium were each separately plated with $5 \times 10^4$ cells of the above same mouse cell line. All of the plated dishes were incubated for 5 days at 37° C. The first group of dishes had an average total cell concentration of $2.4 \times 10^4$, while the second group had an average total cell concentration of $1.2 \times 10^4$.

EXAMPLE 5

Five (5) 35 mm. culture dishes each containing 3 ml. of the fetal calf serum medium described in Example 2 above and also containing 20 micrograms cholesterol (calculated from the cholesterol content of the specific cholesterol-rich fraction prepared in Example 1 above) per ml. of the initial nutrient medium were each separately plated with $5 \times 10^4$ baby hamster kidney cells (ATCC BHK-21). The plated dishes were incubated for 6 days at 37° C. The average total cell concentration was $2 \times 10^5$.

EXAMPLE 6

Five (5) 25 sq. cm. surface area tissue culture flasks each containing 10 ml. of the fetal calf serum nutrient medium described in Example 2 above were each separately plated with $1 \times 10^6$ mouse myeloma cells (ATCC P3x63Ag8.653). Five (5) similar flasks each containing 10 ml. of the same medium and also containing 20 micrograms cholesterol (calculated from the cholesterol content of the specific cholesterol-rich fraction prepared in Example 1 above) per ml. of the initial nutrient medium were each separately plated with $1 \times 10^6$ cells of the same mouse cell line. All of the plated flasks were incubated at 37° C. for 6 days. The medium containing the specific cholesterol-rich fraction had an average total cell concentration of $2.5 \times 10^7$, while the medium without the cholesterol had an average total cell concentration of $1.2 \times 10^6$.

The above experimental data clearly show that the use of the specific cholesterol-rich fraction enables cell culture to be effectively carried out in a nutrient medium containing only 0.5 volume percent fetal calf serum. Under the same conditions and additive concentrations, other cholesterol concentrates show no advantage.

The use of the specific cholesterol-rich fraction with a serum-free medium is shown in the following example.

EXAMPLE 7

Five (5) 35 mm. culture dishes each containing 3 ml. of Ventrex HL-1 serum-free medium were each separately plated with $5 \times 10^4$ mouse fibroblast cells (ATCC L929). Five (5) similar dishes each containing 3 ml. of the same HL-1 medium and also containing 20 micrograms cholesterol (calculated from the cholesterol content of the specific cholesterol-rich fraction prepared in Example 1 above) per ml. of the initial nutrient medium were each separately plated with $5 \times 10^4$ cells of the same mouse cell line. All of the plated dishes were incubated for 6 days at 37° C. The medium containing the specific cholesterol-rich fraction had an average total cell concentration of $5.5 \times 10^5$ while the medium without the cholesterol had an average total cell concentration of $6.5 \times 10^4$.

## Claims

1. A process for isolating and purifying a cholesterol-rich fraction from mammalian blood plasma or serum or fraction thereof containing cholesterol which comprises the steps of:

   (a) contacting a liquid cholesterol-containing plasma or serum or fraction thereof with a silica adsorbent to adsorb the cholesterol;

   (b) separating the adsorbed cholesterol from the remaining liquid plasma or serum;

   (c) freezing and thawing the adsorbed cholesterol;

   (d) eluting the adsorbed cholesterol at a pH from 9.0 to 11.5;

   (e) concentrating the eluted cholesterol solution by ultrafiltration;

   (f) adjusting the pH of the concentrated cholesterol solution to a value in the range from 11.0 to 11.4;

   (g) dialyzing the concentrated cholesterol solution sequentially against sodium carbonate and water;

   (h) further concentrating the dialyzed cholesterol solution by ultrafiltration to desired cholesterol level;

   (i) adjusting the pH of the concentrated cholesterol solution to a value in the range from 7.0 to 11.0;

   (j) heating the concentrated cholesterol solution at 50° to 100° C. for 30 minutes to 24 hours;

   (k) recovering therefrom a purified cholesterol-rich fraction.

2. A process according to Claim 1 employing bovine plasma or bovine serum as the source of cholesterol.

3. A process for the growth of mammalian cells in an appropriate nutrient medium either free of fetal calf serum or containing a significantly reduced level of fetal calf serum wherein such nutrient medium contains the specific cholesterol-rich fraction obtained by the process according to Claims 1 or 2.

4. A process according to Claim 3 wherein the nutrient medium contains from 0.5 to 2.5 volume percent fetal calf serum.

5. A process according to Claims 3 or 4 wherein the cholesterol-rich fraction is obtained from bovine serum or bovine plasma.

6. A process according to any of Claims 3, 4 or 5 wherein the cholesterol-rich fraction is present in the nutrient medium in an amount from 0.0005 to 0.01 percent weight/volume basis cholesterol calculated from the cholesterol content of the cholesterol-rich fraction.

7. A process according to any of Claims 3, 4, 5 or 6 wherein the mammalian cells are mouse fibroblast cells, baby hamster kidney cells, or mouse myeloma cells.

## Revendications

1. Procédé pour isoler et purifier une fraction, riche en cholestérol, de plasma ou de sérum sanguin de mammifères, ou d'une fraction de ce plasma ou sérum, contenant du cholestérol, qui comprend les étapes consistant :

   (a) à mettre en contact un plasma ou sérum liquide, ou une de ses fractions, contenant du cholestérol avec un adsorbant à base de silice pour provoquer l'adsorption du cholestérol ;

   (b) à séparer le cholestérol adsorbé du plasma ou du sérum liquide restant ;

   (c) à congeler et à décongeler le cholestérol adsorbé ;

   (d) à éluer le cholestérol adsorbé à un pH de 9,0 à 11,5 ;

   (e) à concentrer par ultrafiltration la solution de cholestérol éluée ;

   (f) à ajuster le pH de la solution de cholestérol concentrée à une valeur comprise dans la plage de 11,0 à 11,4 ;

   (g) à dialyser la solution de cholestérol concentrée successivement contre du carbonate de sodium et de l'eau ;

   (h) à concentrer davantage par ultrafiltration, à la teneur en cholestérol désirée, la solution de cholestérol dialysée ;

   (i) à ajuster le pH de la solution de cholestérol concentrée à une valeur comprise dans la plage de 7,0 à 11,0 ;

   (j) à chauffer la solution de cholestérol concentrée à une température de 50° à 100° C pendant un temps de 30 minutes à 24 heures ;

   (k) à séparer de cette solution une fraction purifiée, riche en cholestérol.

2. Procédé suivant la revendication 1, utilisant du plasma de boeuf ou du sérum de boeuf comme source de cholestérol.

3. Procédé destiné à faire croître des cellules de mammifère dans un milieu nutritif approprié dépourvu de sérum de veau foetal ou contenant une très basse teneur en sérum de veau foetal, dans lequel ce milieu nutritif contient la fraction spécifique riche en cholestérol obtenue par le procédé suivant la revendication 1 ou 2.

4. Procédé suivant la revendication 3, dans lequel le milieu nutritif contient 0,5 à 2,5 % en volume de sérum de veau foetal.

5. Procédé suivant la revendication 3 ou 4, dans lequel la fraction riche en cholestérol est obtenue à partir de sérum de boeuf ou de plasma de boeuf.

6. Procédé suivant l'une quelconque des revendications 3, 4 et 5, dans lequel la fraction riche en cholestérol est présente dans le milieu nutritif en une quantité de 0,0005 à 0,01 % (en poids/volume) de cholestérol, le calcul étant effectué à partir de la teneur en cholestérol de la fraction riche en cholestérol.

7. Procédé suivant l'une quelconque des revendications 3, 4, 5 et 6, dans lequel les cellules de mammifère sont des fibroblastes de souris, des cellules de rein d'hamster nouveau-né ou des cellules de myélome de souris.

**Patentansprüche**

1. Verfahren zur Isolierung und Reinigung einer cholesterolreichen Fraktion aus Säugetierblutplasma oder -serum oder einer cholesterolhaltigen Fraktion davon, wobei man:

   (a) ein flüssiges cholesterolhaltiges Plasma oder Serum oder eine Fraktion davon mit einem Kieselgeladsorbens zusammenbringt, um das Cholesterol zu adsorbieren;

   (b) das adsorbierte Cholesterol aus dem verbleibenden flüssigen Plasma oder Serum abtrennt;

   (c) das adsorbierte Cholesterol einfriert und wieder auftaut;

   (d) das adsorbierte Cholesterol bei einem pH von 9,0 bis 11,5 eluiert;

   (e) die eluierte Cholesterollösung durch Ultrafiltration aufkonzentriert;

   (f) den pH der aufkonzentrierten Cholesterollösung auf einen Wert im Bereich von 11,0 bis 11,4 einstellt;

   (g) die aufkonzentrierte Cholesterollösung sequentiell gegen Natriumcarbonat und Wasser dialysiert;

   (h) die dialysierte Cholesterollösung durch Ultrafiltration auf den gewünschten Cholesterolgehalt weiter aufkonzentriert;

   (i) den pH der aufkonzentrierten Cholesterollösung auf einen Wert im Bereich von 7,0 bis 11,0 einstellt;

   (j) die aufkonzentrierte Cholesterollösung bei 50 bis 100°C 30 min bis 24 h lang erhitzt;

   (k) und daraus eine gereinigte cholesterolreiche Fraktion gewinnt.

2. Verfahren gemäß Anspruch 1, wobei man Rinderplasma oder Rinderserum als Quelle des Cholesterols einsetzt.

3. Verfahren zum Wachstum von Säugetierzellen in einem geeigneten Nährmedium, das entweder frei von fetalem Kälberserum ist oder einen signifikant verminderten Gehalt an fetalem Kälberserum enthält, wobei dieses Nährmedium die spezifische cholesterolreiche Fraktion, erhältlich durch das Verfahren gemäß der Ansprüche 1 oder 2, enthält.

4. Verfahren gemäß Anspruch 3, wobei das Nährmedium von 0,5 bis 2,5 Volumen-% fetales Kälberserum enthält.

5. Verfahren gemäß der Ansprüche 3 oder 4, wobei die cholesterolreiche Fraktion aus Rinderserum oder Rinderplasma erhalten wird.

6. Verfahren gemäß jedem der Ansprüche 3, 4 oder 5, wobei die cholesterolreiche Fraktion in dem Nährmedium in einer Menge von 0,0005 bis 0,01 % Cholesterol auf Gewicht/Volumen-Basis, berechnet aus dem Cholesterolgehalt der cholesterolreichen Fraktion, vorliegt.

7. Verfahren gemäß jedem der Ansprüche 3, 4, 5 oder 6, wobei die Säugetierzellen Mäuse-Fibroblastzellen, Babyhamsternierenzellen oder Mäuse-Myelomazellen sind.